(19) **Europäisches Patentamt / European Patent Office / Office européen des brevets**

(11) **EP 2 052 718 A1**

(12) **EUROPEAN PATENT APPLICATION**

(43) Date of publication:
29.04.2009 Bulletin 2009/18

(51) Int Cl.:
*A61K 9/70* (2006.01)    *A61K 47/10* (2006.01)
*A61K 47/32* (2006.01)

(21) Application number: 08253376.1

(22) Date of filing: 17.10.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(30) Priority: 19.10.2007 JP 2007272166

(71) Applicant: Nitto Denko Corporation
Ibaraki-shi, Osaka 567-8680 (JP)

(72) Inventors:
• Inosaka, Keigo
Ibaraka-shi, Osaka 567-8680 (JP)
• Tamura, Kei
Ibaraka-shi, Osaka 567-8680 (JP)
• Yoshino, Osamu
Ibaraka-shi, Osaka 567-8680 (JP)

(74) Representative: Duckworth, Timothy John
J.A. Kemp & Co.,
14 South Square,
Gray's Inn
London WC1R 5JJ (GB)

(54) **Patch preparation**

(57) The present invention aims at provision of a patch preparation having a small area and permitting adhesion for a long time.

The present invention provides a patch preparation having an adhesive layer on at least one surface of a support, wherein the adhesive layer contains an adhesive, a poorly soluble drug, a first organic liquid component, a second organic liquid component, a third organic liquid component and crosslinked polyvinylpyrrolidone, the solubility of the poorly soluble drug in the first organic liquid component is not less than the solubility of the poorly soluble drug in the second organic liquid component, and the solubility of the poorly soluble drug in the second organic liquid component is not less than the solubility of the poorly soluble drug in the third organic liquid component.

FIG. 1

EP 2 052 718 A1

**Description**

**TECHNICAL FIELD OF THE INVENTION**

The present invention relates to a patch preparation having an adhesive layer provided on at least one surface of the support.

**BACKGROUND OF THE INVENTION**

Patch preparation drew attention as a superior administration mode offering many advantages such as absorbability of drug in the gastrointestinal tract and avoidance of the first pass effect of drug in the liver, superiority for those having difficulty in swallowing drugs, prevention of forgotten administration and the like, and various kinds of patch preparations have been developed.

Improvement of QOL (Quality of life) drawing attention in these years is considered to be necessary also for patch preparations. Therefore, it is desired to decrease the area of the patch preparation to reduce stress during adhesion, and enable adhesion for a long time to reduce trouble of changing the patch and skin irritation. The related prior art is as follows.

JP-A-8-104625 discloses a patch preparation containing polyvalent alcohol, fatty acid ester of polyvalent alcohol and crosslinked polyvinylpyrrolidone, but does not disclose use of monovalent alcohol.

JP-B-2503095 discloses a patch preparation containing alcohol, fatty acid ester of polyvalent alcohol and crosslinked polyvinylpyrrolidone, but does not disclose use of monovalent alcohol and polyvalent alcohol.

JP-B-7-98744 discloses a patch preparation containing fatty acid ester of polyvalent alcohol, monovalent alcohol and crosslinked polyvinylpyrrolidone, but does not disclose use of polyvalent alcohol.

In addition, the aforementioned prior art does not disclose a fatty acid ester of polyvalent alcohol, which contains at least one hydroxyl group of the polyvalent alcohol, where reduction of the area of patch preparation and long time adhesion have not been achieved at a satisfactory level.

**Disclosure of the Invention**

**Problems to be Solved by the Invention**

In view of the above, the present invention aims at provision of a patch preparation having a small area and permitting adhesion for a long time.

**Means of Solving the Problems**

In one aspect, therefore, the present invention provides a patch preparation having an adhesive layer on at least one surface of a support, wherein the adhesive layer comprises an adhesive, a poorly soluble drug, a first organic liquid component, a second organic liquid component, a third organic liquid component and a crosslinked polyvinylpyrrolidone, the solubility of the poorly soluble drug in the first organic liquid component is not less than the solubility of the poorly soluble drug in the second organic liquid component, and the solubility of the poorly soluble drug in the second organic liquid component is not less than the solubility of the poorly soluble drug in the third organic liquid component.

Preferably, in the present invention, the first organic liquid component is a first polyvalent alcohol, the second organic liquid component is a fatty acid ester of a second polyvalent alcohol having at least one hydroxyl group of the second polyvalent alcohol, and the third organic liquid component is a monovalent alcohol.

Preferably, in the present invention, the fatty acid ester of the second polyvalent alcohol is a fatty acid monoester of a divalent alcohol, or a fatty acid mono- or diester of a trivalent alcohol.

Preferably, in the present invention, the adhesive layer contains 0.1 - 15 parts by weight of the second organic liquid component relative to 100 parts by weight of the adhesive.

Preferably, in the present invention, the adhesive is a rubber adhesive.

Preferably, in the present invention, the poorly soluble drug has a melting point of not less than 100°C.

**Effect of the Invention**

Crosslinked polyvinylpyrrolidone contained in the adhesive layer of the patch preparation of the present invention can retain the first organic liquid component capable of dissolving a large amount of a poorly soluble drug. In addition, the first organic liquid component enables the adhesive layer to stably retain the drug in a dissolution state before adhesion even when the component has low compatibility with an adhesive elastomer. Therefore, since the patch preparation of the present invention can retain a poorly soluble drug at a high concentration in the adhesive layer, it can retain a sufficient amount of the poorly soluble drug in a small area while suppressing crystallization of the drug.

When the patch preparation of the present invention is adhered to the skin, crosslinked polyvinylpyrrolidone in the adhesive layer may attract water from the skin such as sweat and the like to the adhesive layer, and the attracted water may preferentially release the first organic liquid component having high solubility of the poorly soluble drug from the adhesive layer. As a result, the poorly soluble drug may also be released from the adhesive layer. Consequently, the patch preparation of the present invention is superior in the releaseability of the poorly soluble drug in the early stage of adhesion to the skin.

Since the first organic liquid component having higher solubility of the poorly soluble drug as compared to the second organic liquid component may preferentially be released from the adhesive layer in the early stage of adhesion to the skin, the second organic liquid component having lower solubility of the poorly soluble drug as compared to the first organic liquid component may preferentially remain in the adhesive layer. Consequently, the solubility of the poorly soluble drug in the adhesive layer that has become a new liquid component composition decreases, the poorly soluble drug may be divided between the adhesive layer and the stratum corneum layer, and released well. Therefore, the poorly soluble drug may stably be released in a sustained manner at a high level still in the middle stage of adhesion to the skin.

Since the second organic liquid component having higher solubility of the poorly soluble drug as compared to the third organic liquid component may preferentially be released from the adhesive layer in the middle stage of adhesion to the skin, the third organic liquid component having lower solubility of the poorly soluble drug as compared to the second organic liquid component may preferentially remain in the adhesive layer. Consequently, the solubility of the poorly soluble drug in the adhesive layer that has become a new liquid composition may decrease further, the poorly soluble drug may be divided between the adhesive layer and the stratum corneum layer, and released well. Therefore, the poorly soluble drug may stably be released in a sustained manner at a high level still in the late stage of adhesion to the skin.

In the manner described above, the patch preparation of the present invention enables adhesion for a long time even when it has a small area.

## BRIEF DESCRIPTION OF THE DRAWINGS

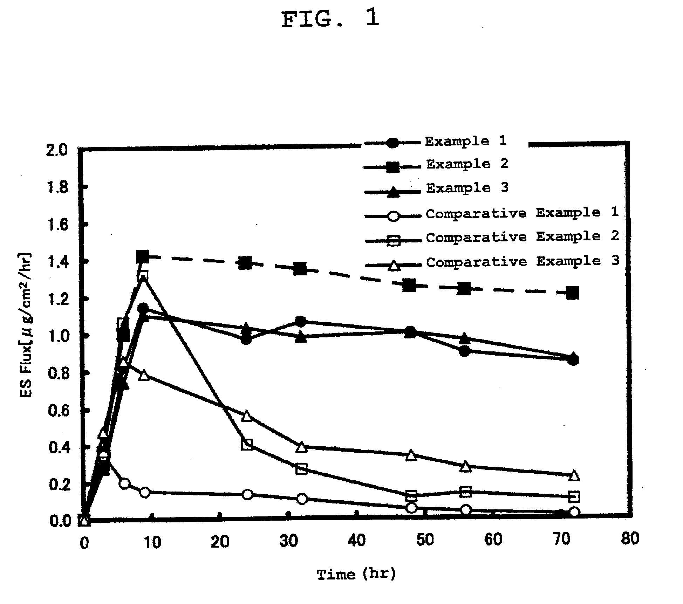

Fig. 1 shows the permeability test results in the Examples and Comparative Examples.

## Best Mode for Carrying out the Invention

Preferable embodiments of the present invention are shown below. However, the detailed explanation thereof and particular examples thereof are exclusively intended for exemplification purposes, and do not limit the scope of the present invention. The following explanation of the preferable embodiments is exclusively intended for exemplification purposes, and does not intend to limit the present invention, application thereof and use thereof.

While a material for the support to be used in the present invention is not particularly limited, a preferable material is one that does not allow additives and poorly soluble drugs to pass through the support and the back face and lost from the back face, which decreases their contents. Namely, a material substantially impermeable to these components is preferable. Specifically, films made from polyester, nylon, polyvinyl chloride, polyethylene, polypropylene, ethylene-vinyl acetate copolymer, polytetrafluoroethylene, ionomer resin etc., metal foil, laminate films thereof and the like are available. Among these, to improve, as a support, the adhesiveness (anchor property) to the adhesive layer, it is preferable to comprise, as a support, a laminate film of a non-porous film made of the above-mentioned material and a porous film and form an adhesive layer on the porous film side.

The porous film is not particularly limited as long as the adhesive layer has good anchor property. For example, paper, woven fabric, non-woven fabric, mechanically perforated sheet and the like can be used. Particularly, paper, woven fabric and non-woven fabric are preferable. The thickness of the porous film is 10 - 500 $\mu$m to improve anchor force and flexibility of a patch preparation, and about 10 - 200 $\mu$m for a thin patch preparation such as plaster type and adhesive tape type preparations. The fabric weight of woven fabric and non-woven fabric is preferably 5 - 30 g/m$^2$ to improve anchor force.

The patch preparation of the present invention has an adhesive layer on at least one surface of the support. The adhesive layer contains an adhesive. For skin adhesiveness, the adhesive is substantially of a type free of water, namely, a hydrophobic adhesive is preferable. While the adhesive is not particularly limited, acrylic adhesives, rubber adhesives, vinyl ether adhesives, vinyl ester adhesives and polyester adhesives are available, which are used alone or in a combination of two or more kinds thereof.

While the weight of the adhesive (total weight when two or more kinds are blended) is not particularly limited, it is preferably 30 - 92.5 wt%, more preferably 50 - 90 wt%, most preferably 70 - 80 wt%, of the total weight of the adhesive layer.

From the aspects of property of adhesiveness to the skin, particularly adhesiveness in the early stage or perspiration resistance, acrylic adhesives, inter alia, copolymers obtained by copolymerization of (meth)acrylic acid alkyl ester as a main component are preferable. Specific examples of (meth)acrylic acid alkyl ester include (meth)acrylic acid alkyl esters wherein the alkyl group is a straight chain or branched alkyl group having 4 or more carbon numbers such as butyl, pentyl, hexyl, heptyl, octyl, nonyl, decyl, undecyl, dodecyl, tridecyl and the like, which can be used alone or a combination of two or more kinds thereof.

Examples of the monomer copolymerizable with the above-mentioned (meth)acrylic acid alkyl ester include monomers having a carboxyl group such as (meth)acrylic acid, itaconic acid, maleic acid, maleic anhydride and the like; monomers having sulfonic acid group such as styrenesulfonic acid, allylsulfonic acid, sulfopropyl(meth)acrylate, (meth) acryloyloxynaphthalenesulfonic acid, acrylamidemethylsulfonic acid and the like; monomers having a hydroxyl group such as (meth)acrylic acid 2-hydroxyethylester, hydroxypropyl (meth)acrylate and the like; (meth)acrylic acid derivatives having an amide group such as (meth)acrylamide, dimethyl(meth)acrylamide, N-butyl(meth)acrylamide, N-methylol (meth)acrylamide and the like; (meth)acrylic acid aminoalkyl esters such as aminoethyl (meth)acrylate, dimethylaminoethyl (meth)acrylate, t-butylaminoethyl (meth)acrylate and the like; (meth)acrylic acid alkoxy esters such as methoxyethyl (meth)acrylate, ethoxyethyl (meth)acrylate, tetrahydrofurfuryl (meth)acrylate and the like; alkoxyalkyleneglycol (meth)acrylates such as methoxyethyleneglycol (meth)acrylate, methoxydi(ethylene glycol) (meth)acrylate, methoxypoly (ethylene glycol) (meth)acrylate, methoxypoly(propylene glycol) (meth)acrylate and the like; compounds having vinyl group such as (meth)acrylonitrile; vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole, vinylmorpholine and the like; and the like, which can be used alone or a combination of two or more kinds thereof. Copolymerization of these monomers can be appropriately set according to the weight average molecular weights of the obtained copolymers.

Of these, from the aspects of the adhesion force and cohesion strength that the copolymers themselves have, for example, a copolymer of 2-ethylhexyl acrylate, N-vinyl-2-pyrrolidone and acrylic acid, a copolymer of 2-ethylhexyl acrylate, 2-hydroxyethyl acrylate and vinyl acetate and the like are preferable.

As the adhesive, a non-acrylic adhesive is preferable from the aspects of sufficient hydrophobicity and skin adhesion force. The non-acrylic adhesive is a composition has adhesiveness afforded by a non-acrylic elastomer and the below-mentioned tackifier. Of such non-acrylic adhesives, from the aspects of easiness to obtain, for example, adhesives containing rubber elastomers, for example, elastomers such as styrene-diene-styrene block copolymers (e.g., styreneisoprene-styrene block copolymer, styrene-butadiene-styrene block copolymer and the like), polyisoprene, polyisobutylene, polybutadiene and the like; silicone elastomers such as silicone rubber, dimethylsiloxane base, diphenylsiloxane base and the like; vinyl ether elastomers such as poly(vinyl methyl ether), poly(vinyl ethyl ether), poly(vinyl isobutyl ether) and the like; vinyl ester elastomers such as ethylene-vinyl acetate copolymer and the like; polyester elastomers made of a carboxylic acid component such as dimethylterephthalate, dimethylisophthalate, dimethylphthalate and the like and a polyvalent alcohol component such as ethylene glycol and the like; and the like are preferable, which can be used alone or a combination of two or more kinds thereof.

Of these, a rubber adhesive containing a rubber elastomer, particularly an adhesive containing polyisobutylene, is preferable to achieve the balance of adhesive property, stability and safety. Depending on the viscosity average molecular weight, the rubber elastomer can be used alone or in a combination of two or more kinds. When a rubber adhesive is used alone, a rubber elastomer to be essentially used is referred to as a first rubber elastomer, and rubber elastomers to be further added depending on the object are referred to as a second rubber elastomer, a third rubber elastomer and the like.

While the viscosity average molecular weight of the first rubber elastomer is not particularly limited, it is preferably 1,600,000 - 6,500,000, more preferably, 2,000,000 - 6,000,000, still more preferably 2,500,000 - 5,500,000, most preferably 3,000,000 - 5,000,000.

When the viscosity average molecular weight of the first rubber elastomer is not less than 1,600,000, the adhesive layer can easily retain a large amount of an organic liquid component, since the molecular chain thereof is sufficiently long and intricately entangled. When the viscosity average molecular weight of the first rubber elastomer is not more than 6,500,000, it is easy to balance the adhesion properties such as tackiness and the like, in combination with other components.

The viscosity average molecular weight in the present specification is determined by calculating the Staudinger index ($J_0$) by the Schulz-Blaschke equation using the flow time of capillary 1 in a Ubbelohde viscosimeter at 20°C, and from the following formula using the $J_0$ value:

$$J_0 = \eta_{sp}/c(1+0.31\eta_{sp})\,\mathrm{cm}^3/\mathrm{g}\quad(\text{Schulz-Blaschke equation})$$

$$\eta_{sp}=t/t_0-1$$

t: flow time of solution (by Hagenbach-Couette Correction) to: flow time of solvent (by Hagenbach-Couette Correction) c: concentration of solution ($g/cm^3$)

$$J_0=3.06\times10^{-2}\ \overline{Mv}^{\ 0.65}$$

$\overline{Mv}$: viscosity average molecular weight If desired, the adhesive layer may further contain a second rubber elastomer having a viscosity average molecular weight of 40,000 - 85,000. Concurrently using the second rubber elastomer having high flowability as compared to the first rubber elastomer, separation of the first rubber elastomer from the other adhesive layer components can be prevented, and the adhesive layer can have adequate flexibility.

When the viscosity average molecular weight of the second rubber elastomer is less than 40,000, the affinity of the second rubber elastomer and the other adhesive layer components becomes high and the affinity of the other adhesive layer components and the first rubber elastomer becomes low, possibly resulting in the separation of these. In contrast, when the viscosity average molecular weight of the second rubber elastomer is more than 85,000, the affinity of the first rubber elastomer component and the second rubber elastomer becomes high, and the affinity of the other adhesive layer component and the second rubber elastomer becomes low, possibly resulting in the separation of these.

The second rubber elastomer is independently selected from those similar to the first rubber elastomer. The first rubber elastomer and the second rubber elastomer may be of the same kind or different kinds. They are preferably of the same kind in view of the compatibility between them.

While the proportion of the second rubber elastomer in the adhesive layer is not particularly limited, it is within the range of preferably 50 - 200 parts by weight, more preferably 50 - 150 parts by weight and most preferably 90 - 110 parts by weight, relative to 100 parts by weight of the first rubber elastomer. When the amount of the second rubber elastomer is less than 50 parts by weight, only the property of the first rubber elastomer may be expressed. In contrast, when the amount of the second rubber elastomer is more than 200 parts by weight, the cohesion strength of the adhesive layer may decrease.

Alternatively, a second rubber elastomer having a viscosity average molecular weight higher than that of the first rubber elastomer is preferable, if the second fubber elastomer is added for the purpose of further enhancing the cohesion strength of the adhesive layer. A third rubber elastomer may optionally be added to the adhesive layer.

The adhesive layer contains a poorly soluble drug. Here, the poorly soluble drug means a drug which cannot be dissolved, in the adhesive layer, easily in an amount sufficient to administer an effective amount thereof to the target. More specifically, a drug having the coefficient of partition (1-octanol/water), i.e., log Pow, of 0.5 - 5.5, and a melting point of not less than 100°C. To sufficiently achieve the effect of the present invention, log Pow of the poorly soluble drug is preferably 1.0 - 5.0, more preferably 3.0 - 5.0. While the upper limit value of the melting point is not particularly limited, it is preferably not more than 300°C for practical reasons.

When the log Pow of the drug is less than 0.5, since the hydrophilicity of such drug is high, a crystal of the drug may precipitate in the adhesive layer even in the present invention. When the log Pow of the drug is more than 5.5, since the hydrophobicity of such drug is high, it is highly unlikely a crystal of the drug will precipitate in the adhesive layer, where the advantage of the present invention is not high.

The log Pow here is an index showing the hydrophilicity or hydrophobicity of a drug, and refers to a value obtained for each drug by a measurement according to the method described in "OECD GUIDELINE FOR THE TESTING OF CHEMICALS 107, Adopted by the Council on 27th July 1995, Partition Coefficient (n-octanol/water), Shake Flask Method", where the base of the logarithm is 10. In this embodiment, log Pow is calculated using a computational software Cache (registered trademark) for log Pow, manufactured by FUJITSU LTD. For measurement (calculation) of log Pow, a structural formula of a compound is input in the aforementioned computational software for calculation.

The melting point of the drug here means a value measured by the following method.
Apparatus: melting point measurement apparatus manufactured by MIYAMOTO RIKEN IND JAPAN Measurement method: According to the first method of the melting point measurement method in the Japanese Pharmacopoeia, the reading on a temperature gauge at which a sample is liquefied in the capillary and does not show a solid at all is taken as the melting point.

The poorly soluble drug is not particularly limited, and is preferably one that can be administered to a mammal such as human and the like through the skin, i.e., a transdermally absorbable poorly soluble drug. Specific examples of such drug include general anesthetics, hypnotic sedatives, antiepileptic drugs, antipyretic analgesic antiphlogistic drugs, anti-vertigenous drugs, psychoneurotic drugs, topical anesthetics, skeleton muscle relaxants, autonomic drugs, antiepileptic drugs, anti-parkinsonian drugs, anti-histamine drugs, cardiac stimulants, drugs for arrhythmia, diuretics, hypotensive drugs, vasoconstrictors, coronary vasodilators, peripheral vasodilators, arteriosclerosis drugs, drugs for circulatory organs, anapnoics, antitussive expectorants, hormone drugs, external drugs for mattery diseases, analgesic·antipruritic· styptic·antiphogistic drugs, drugs for parasitic dermatic diseases, drugs for arrest of bleeding, gout treatment drugs, drugs for diabetes, drugs for anti-malignant tumor, antibiotics, chemical therapy drugs, narcotics, quit smoking aids and the like.

Among the poorly soluble drugs, estradiol (female hormone) is a drug used for the treatment of climacteric disorder. Estradiol is a substance having biological properties similar to those of estradiol, and shows similar physicochemical properties. Examples of estradiol include egg-yolk hormones such as estradiol (4.008; melting point 179°C), estrin (4.535; melting point 256°C), estriol (3.239; melting point 282°C), ethynylestradiol (estrogen, 4.017; melting point 146°C, hemi), norelgestromin (progestin, 3.689; melting point 131°C) and the like. The log Pow and melting point of them are shown in the parentheses.

Climacteric disorder has recently been reported to occur not only in women but also in men. The subject is not less than 50% of the population. The present invention took note of this disorder since it poses serious problems, though at varying levels, for the improvement of QOL (Quality of life) attracting attention in recent years. It is also true that this disorder is not properly recognized as a disease in Japan where the recognition prevails that it is not a disease but something everyone goes through.

Possibly due to such recognition, the target patients do not have a negative image of a disease, and most patients lead a daily life without any change. Therefore, even though an estradiol-containing patch preparation is particularly required to have a reduced area and be able to be adhered for a long time. Estradiol is a particularly effective drug for application to the patch preparation of the present invention since it is poorly soluble in the adhesive layer.

The proportion of the poorly soluble drug in the adhesive layer is not particularly limited as long as the effect of the poorly soluble drug can be exerted and the adhesive property of the adhesive is not impaired. It is preferably 0.01 - 5 wt%, more preferably 0.2 - 3 wt%, based on the total weight of the adhesive layer. When the weight is less than 0.01 wt%, the treatment effect may not be sufficient, and when it is more than 5 wt%, skin irritation and precipitation in the adhesive layer may occur.

In the present invention, at least three kinds of organic liquid components are contained in the adhesive layer. Such organic liquid components enables continuous and stable release of the poorly soluble drug for a long time while maintaining a high release speed. As mentioned above, the at least three kinds of organic liquid components are hereinafter referred as the first organic liquid component, the second organic liquid component and the third organic liquid component. These organic liquid components satisfy the following relationship: the solubility of the poorly soluble drug in the first organic liquid component is not less than, preferably more than, the solubility of the poorly soluble drug in the second organic liquid component, and the solubility of the poorly soluble drug in the second organic liquid component is not less than, preferably more than, the solubility of the poorly soluble drug in the third organic liquid component. In the present specification, the solubility of the poorly soluble drug in the organic liquid components means a weight (mg) of a drug dissolved in the organic liquid components (1 g) at room temperature (25°C) in 24 hr as measured by HPLC.

While the first organic liquid component is not particularly limited as long as it satisfies the aforementioned relationship with the second and the third organic liquid components, it is preferably selected from the first polyvalent alcohol so that the relationship can be satisfied. Since the poorly soluble drug is a compound having a good balance between hydrophilicity and hydrophobicity at a certain level, the first organic liquid component that dissolves the drug is desired to have a moderate balance between hydrophilicity and hydrophobicity. Accordingly, the first organic liquid component is preferably the first polyvalent alcohol. Specific examples of the first polyvalent alcohol include glycol such as ethylene glycol, di(ethylene glycol), tri(ethylene glycol), poly(ethylene glycol), propylene glycol, di(propylene glycol), poly(propylene glycol) and the like. These are used alone or in a mixture of two or more kinds. More preferable examples of the first polyvalent alcohol include propylene glycol and di(propylene glycol).

The amount of the first organic liquid component to be used is within the range of preferably 1 - 15 parts by weight, more preferably 2 - 10 parts by weight, relative to 100 parts by weight of the adhesive.

The second organic liquid component is selected in such a manner that the above-mentioned relationship will be satisfied, and specific preferable examples thereof include fatty acid esters of the second polyvalent alcohol. The second polyvalent alcohol may be the same as or different from the first polyvalent alcohol, and is preferably different since the solubility of the poorly soluble drug can be improved.

In the present invention, the aforementioned fatty acid ester of the second polyvalent alcohol has at least one hydroxyl group of the second polyvalent alcohol. Examples of such ester include an ester wherein the hydroxyl group in the number of 1 to (n-1) from the hydroxyl groups in the number of n of an n-valent alcohol, which is a second polyvalent alcohol, is ester bonded with the carboxyl group of a fatty acid. More specifically, an ester wherein one or two hydroxyl groups of three hydroxyl groups of a trivalent alcohol is(are) ester bonded with the carboxyl group of a fatty acid, and an ester wherein one hydroxyl group of two hydroxyl groups of a divalent alcohol is ester bonded with the carboxyl group of a fatty acid and the like can be given as examples. Since such organic liquid component has both a hydrophilic part and a hydrophobic part in a molecule, the second organic liquid component is suitable for the object of the present invention. From the aspect of the balance of hydrophilic part and hydrophobic part, an ester wherein one hydroxyl group of the second polyvalent alcohol is ester bonded with a fatty acid, that is, a monoester is preferable.

Examples of such second polyvalent alcohol fatty acid ester include propylene glycol monolaurate, propylene glycol monostearate, propylene glycol fatty acid ester (a.k.a.: miglyol) and stearic acid glycol, which are used alone or in a mixture of two or more kinds. To maintain the drug solubility of the first organic liquid component and further from the aspects of the balance of promotion of permeation of a poorly soluble drug from the elastomer to the skin and skin irritation, propylene glycol monolaurate is particularly preferable. The amount of the second organic liquid component to be used is within the range of preferably 0.1 - 15 parts by weight, more preferably 1 - 10 parts by weight, relative to 100 parts by weight of the adhesive.

As the third organic liquid component, monovalent alcohol is preferable since it has sufficient hydrophobicity. Specific examples of the monovalent alcohol include oleyl alcohol, geraniol, octyldodecanol, stearyl alcohol, isostearyl alcohol and cetanol, which are used alone or in a mixture of two or more kinds. From the solubility of the poorly soluble drug, a straight chain alcohol rather than a branched alcohol is effective, and oleyl alcohol is more preferable. The amount of the third organic liquid component to be used is within the range of preferably 1 - 20 parts by weight, more preferably 3 - 10 parts by weight, relative to 100 parts by weight of the adhesive.

When the amount of the aforementioned first, second and third organic liquid components to be blended is not less than the lower limit, sufficient solubility of a poorly soluble drug in the adhesive layer can be ensured. When the amount of the aforementioned first, second and third organic liquid components to be blended is not more than the upper limit, the maintenance of these organic liquid components in the adhesive layer can be ensured.

The adhesive layer contains crosslinked polyvinylpyrrolidone. The crosslinked polyvinylpyrrolidone is obtained by copolymerizing N-vinyl-2-pyrrolidone and a multifunctional monomer. Examples of the multifunctional monomer to be used include di(meth)acrylates such as hexamethylene glycol di(meth)acrylate, ethylene glycol di(meth)acrylate, tri (ethylene glycol) di(meth)acrylate and the like; tri(meth)acrylates such as trimethylolpropanetri(meth)acrylate and the like; tetra(meth)acrylates such as pentaerythritoltetra(meth)acrylate and the like; polyallyl compounds such as di(ethylene glycol)bisallylcarbonate, triallylglycerol, triallylcyanurate and the like; polymaleimide compounds such as ethylenebismaleimide etc.; and the like. In addition, divinylbenzene, methylenebisacrylamide, ethylidenebisvinylpyrrolidone, divinylketone, butadiene, isoprene and the like can also be used.

The amount of the multifunctional monomers to be copolymerized is preferably 0.1 - 10 mol% relative to the total amount of the monomers. When the amount is less than 0.1 mol%, the obtained crosslinked polyvinylpyrrolidone is dissolved or swollen in the elastomer, and may be difficult to function as a crosslinked form. When the amount of the multifunctional monomer to be used is more than 10 mol%, the property of vinylpyrrolidone is diluted and exertion of the property may be difficult.

The organic liquid components having comparatively high solubility of poorly soluble drugs tends to show high polarity. Thus, the components easily exude from the adhesive layer. The crosslinked polyvinylpyrrolidone functions to retain such first organic liquid components in the adhesive layer.

Crosslinked polyvinylpyrrolidone is commercially available under a trade name of Kollidon CL, Kollidon CL-M (BASF Japan Ltd.), Poly-Plasdone (ISP Japan Ltd.), Crospovidone (GOKYO TRADING CO., LTD.) and the like. In consideration of the effect relative to the amount of addition thereof, Kollidon CL-M is preferable since particle type small products have widest surface area and are effective. The amount of the crosslinked polyvinylpyrrolidone to be added is within the range of preferably 5 - 40 parts by weight, more preferably 10 - 20 parts by weight, relative to 100 parts by weight of the adhesive.

When desired, the adhesive layer can contain a further organic liquid component to, for example, plasticize the adhesive layer and to decrease skin irritation. Specific examples include fatty acid ester such as diisopropyl adipate, phthalic acid ester, diethyl sebacate, isotridecyl myristate and the like, particularly fatty acid alkyl ester comprising higher fatty acid having a carbon number of 12 to 16 and lower monovalent alcohol having a carbon number of 1 to 4, such as isopropyl myristate, ethyl laurate , ethyl oleate, diisopropyl adipate, diisopropyl palmitate, octyl palmitate and the like; fatty acid having a carbon number of 8 to 10; fats and oils such as olive oil, castor oil, squalene, lanolin and the like; organic solvent such as ethyl acetate, ethyl alcohol, dimethyldecylsulfoxide, methyloctylsulfoxide, dimethyl sulfoxide, dimethylformamide, dimethylacetamide, dimethyllaurylamide, dodecylpyrrolidone, isosorbitol and the like; hydrocarbons such as liquid paraffin and the like; besides them, ethoxylated stearyl alcohol, glycerol ester (in liquid form at room temperature), N-methylpyrrolidone, oleic acid, 1,3-propanediol, glycerol and the like. Needless to say, one in a liquid form at ambient temperature is used from among these. In addition, they are used alone or in a mixture of two or more kinds. To improve adhesiveness by improving the compatibility of the adhesive layer components, fatty acid alkyl ester is preferable. Particularly, a fatty acid ester comprising higher fatty acid having a carbon number of 12 to 16 and lower monovalent alcohol having a carbon number of 1 to 4 is more preferable from the aspect of stability and transdermal absorption-promoting effect.

Furthermore, to improve transdermal absorbability of a poorly soluble drug, fatty acid having a carbon number of 8 to 10 and glycerol may be used in combination with the above-mentioned fatty acid ester. Examples of the fatty acid having a carbon number of 8 to 10 include caprylic acid (octanoic acid, C8), pelargonic acid (nonane acid, C9), capric acid (decane acid, C10) and the like.

The amount of the above-mentioned further organic liquid component to be added is within the range of preferably 1 - 30 parts, more preferably 5 - 10 parts by weight, relative to 100 parts by weight of the adhesive. By setting the amount to not less than 1 part by weight, plasticization of the adhesive layer occurs effectively and skin irritation can be decreased. On the other hand, when the amount is more than 30 parts by weight, the liquid plasticizer may not be maintained in the adhesive layer by the cohesion strength possessed by the adhesive layer, and the liquid plasticizer sometimes bleeds on the surface of the adhesive layer to degrade the adhesiveness.

When the adhesive contains a tackifier, examples of the tackifier include polybutene, rosin resin, terpene resin, petroleum resin, coumarone resin and the like. These may be used alone or in a mixture of two or more kinds. The proportion of the tackifier is within the range of preferably 30 - 90 wt%, more preferably 50 - 70 wt%, based on the total weight of the adhesive. When the proportion of the tackifier is not less than 30 wt%, good tack property can be obtained. On the other hand, when the proportion is more than 90 parts by weight, the adhesive layer sometimes unpreferably shows a destruction tendency.

The adhesive layer may contain, as an optional component, other additives (e.g., surfactant such as glycerol fatty acid ester, sorbitan fatty acid ester etc., organic solvent having high boiling point such as dimethyl sulfoxide, N-methylpyrrolidone etc., absorption promoter such as pyrrolidone carboxylate etc. and the like), other rubber components and the like, as long as the effect of the present invention is not inhibited. The proportion of the additive is preferably 1 - 10 wt% based on the total weight of the adhesive layer except crosslinking agent. The thickness of the adhesive layer is generally 20 - 400 $\mu$m, preferably 30 - 300 $\mu$m.

When desired, the adhesive layer may be subjected to a physical crosslinking treatment by irradiation such as UV or electron beam irradiation and the like, a chemical crosslinking treatment using various crosslinking agents and the like. For a crosslinking treatment without an adverse influence for the drug and the like, a chemical crosslinking treatment by a crosslinking agent is preferable. The crosslinking agent is not particularly limited, but crosslinking agents free of inhibition of crosslink formation by a basic drug, for example, organic metal compounds (e.g., zirconium and zinc alaninate, zinc acetate, glycine ammonium zinc etc.), metal alcoholates (e.g., tetraethyl titanate, tetraisopropyl titanate, aluminum isopropylate, aluminum butyrate etc.), metal chelate compounds (e.g., di-i-propoxybis(acetylacetone)titanate, tetraoctylene glycol titanate, aluminum isopropylate, ethylacetoacetate aluminum diisopropylate, ethylacetoacetate aluminum diisopropylate, aluminum tris(ethyl acetate), aluminum tris(ethyl acetate), aluminum tris(acetyl acetate) etc.) and the like are preferable. These can be used alone or in a mixture of two or more kinds. The amount of the crosslinking agent to be added is within the range of generally 0.1 - 1 part by weight, relative to 100 parts by weight of the adhesive.

The patch preparation of the present invention as mentioned above enables administration of an effective amount of a poorly soluble drug by a small area of preferably 1 - 30 cm$^2$, more preferably 2 - 20 cm$^2$.

**Examples**

The present invention is explained in detail in the following by referring to Examples, which are not to be construed as limitative. In the following Examples, "parts" means "parts by weight".

(Preparation of adhesive A)

The adhesive A was obtained by mixing polyisobutylene (14.7 parts) having a viscosity average molecular weight of 4,000,000 as the first rubber elastomer, polyisobutylene (14.7 parts) having a viscosity average molecular weight of 55,000 as the second rubber elastomer and polybutene (44.2 parts) as a tackifier.

(Poorly soluble drug)

Estradiol was used. Estradiol has a log Pow of 4.008 and a melting point of 179°C.

(Organic liquid component)

di(propylene glycol) as the first organic liquid component: solubility of estradiol 133 mg/g; propylene glycol monolaurate as the second organic liquid component: solubility of estradiol 23 mg/g; oleyl alcohol as the third organic liquid component: solubility of estradiol 12 mg/g; and isopropyl myristate as other organic liquid component: solubility of estradiol 0 mg/g.

<Example 1>

A coating solution was obtained by adding n-hexane to a mixture of adhesive A (73.6 parts), di(propylene glycol) (3 parts), propylene glycol monolaurate (3 parts), oleyl alcohol (5 parts), isopropyl myristate (5 parts) and crosslinked polyvinylpyrrolidone (Kollidon CL-M, manufactured by BASF, 10 parts), and then adding estradiol (0.4 parts). A polyester film (thickness 75 $\mu$m) was coated with the coating solution such that the thickness after drying became 160 $\mu$m, and the coated layer was dried. The coated film was adhered to a polyester film (thickness 12 $\mu$m), and the obtained film was punched out to a piece of 5 cm$^2$ to give the patch preparation of the present invention.

<Example 2>

In the same manner as in Example 1 except that a coating solution was obtained by adding n-hexane to a mixture of adhesive A (71.6 parts), di(propylene glycol) (5 parts), propylene glycol monolaurate (3 parts), oleyl alcohol (5 parts), isopropyl myristate (5 parts) and crosslinked polyvinylpyrrolidone (Kollidon CL-M, manufactured by BASF, 10 parts), and then adding estradiol (0.4 parts), the patch preparation of the present invention was obtained.

<Example 3>

In the same manner as in Example 1 except that a coating solution was obtained by adding n-hexane to a mixture of adhesive A (78.6 parts), di(propylene glycol) (3 parts), propylene glycol monolaurate (3 parts), oleyl alcohol (5 parts) and crosslinked polyvinylpyrrolidone (Kollidon CL-M, manufactured by BASF, 10 parts) and then adding estradiol (0.4 parts), the patch preparation of the present invention was obtained.

<Comparative Example 1>

In the same manner as in Example 1 except that a coating solution was obtained by adding n-hexane to a mixture of adhesive A (76.6 parts), propylene glycol monolaurate (3 parts), oleyl alcohol (5 parts), isopropyl myristate (5 parts) and crosslinked polyvinylpyrrolidone (Kollidon CL-M, manufactured by BASF, 10 parts), and then adding estradiol (0.4 parts), the patch preparation of Comparative Example 1 was obtained.

<Comparative Example 2>

In the same manner as in Example 1 except that a coating solution was obtained by adding n-hexane to a mixture of adhesive A (76.6 parts), di(propylene glycol) (3 parts), oleyl alcohol (5 parts), isopropyl myristate (5 parts) and crosslinked polyvinylpyrrolidone (Kollidon CL-M, manufactured by BASF, 10 parts), and then adding estradiol (0.4 parts), the patch preparation of Comparative Example 2 was obtained.

<Comparative Example 3>

In the same manner as in Example 1 except that a coating solution was obtained by adding n-hexane to a mixture of adhesive A (78.6 parts), di(propylene glycol) (3 parts), propylene glycol monolaurate (3 parts), isopropyl myristate (5 parts) and crosslinked polyvinylpyrrolidone (Kollidon CL-M, manufactured by BASF, 10 parts), and then adding estradiol (0.4 parts), the patch preparation of Comparative Example 3 was obtained.

<Comparative Example 4>

In the same manner as in Example 1 except that a coating solution was obtained by adding n-hexane to a mixture of adhesive A (83.6 parts), di(propylene glycol) (3 parts), propylene glycol monolaurate (3 parts), oleyl alcohol (5 parts) and isopropyl myristate (5 parts), and then adding estradiol (0.4 parts), the patch preparation of Comparative Example 4 was obtained. The compositions of the adhesive layers of Examples and Comparative Examples as mentioned above are shown in Table 1.

Table 1

| unit (parts) | Ex. 1 | Ex. 2 | Ex. 3 | Com. Ex. 1 | Com. Ex. 2 | Com. Ex. 3 | Com. Ex. 4 |
|---|---|---|---|---|---|---|---|
| adhesive A | 73.6 | 71.6 | 78.6 | 76.6 | 76.6 | 78.6 | 83.6 |
| di(propylene glycol) | 3 | 5 | 3 | | 3 | 3 | 3 |

(continued)

| unit (parts) | Ex. 1 | Ex. 2 | Ex. 3 | Com. Ex. 1 | Com. Ex. 2 | Com. Ex. 3 | Com. Ex. 4 |
|---|---|---|---|---|---|---|---|
| propylene glycol monolaurate | 3 | 3 | 3 | 3 | | 3 | 3 |
| oleyl alcohol 5 | | 5 | 5 | 5 | 5 | | 5 |
| isopropyl myristate | 5 | 5 | 5 | 5 | 5 | 5 | 5 |
| crosslinked polyvinylpyrrolidone | 10 | 10 | 10 | 10 | 10 | 10 | |
| estradiol | 0.4 | 0.4 | 0.4 | 0.4 | 0.4 | 0.4 | 0.4 |
| total | 100 | 100 | 100 | 100 | 100 | 100 | 100 |

(Experimental Examples) The patch preparations obtained in the above-mentioned Examples or Comparative Examples were subjected to the following test.

Experimental Example 1 (Adhesion force test)

(Test method)

Adhesion under JIS-Z0237, a measurement was performed using a stainless plate (a test piece of width 12 mm). The results are shown in Table 2 and Table 3. The measured values are directly shown.
Table 2

| - | Ex. 1 | Ex. 2 | Ex. 3 |
|---|---|---|---|
| adhesion force (N/12 mm) | 2.5 | 2.0 | 1.5 |

Table 3

| - | Com. Ex. 1 | Com. Ex. 2 | Com. Ex. 3 | Com. Ex. 4 |
|---|---|---|---|---|
| adhesion force (N/12 mm) | 4.1 | 3.3 | 2.6 | Interface destruction |

In Comparative Example 4, the organic component having high polarity could not be retained in the adhesive layer, and bled on the surface, and therefore, the adhesion force could not be measured. This was considered to be attributable to the fact that the adhesive layer did not contain crosslinked polyvinylpyrrolidone. Adhesiveness and long-term adhesiveness of the other samples were considered to be free of problems.
Experimental Example 2 (Permeability test)

(Test method)

For evaluation of the skin permeability of a drug, the permeability of a drug through the skin of a hairless mouse (male, 8-week-old) was evaluated using a membrane permeation test apparatus (manufactured by VIDREX). The test temperature was 32°C. The solution in the container was collected at intervals of a given time, and the same amount of a receptor fluid (saline) at 32°C was added.
The operation was repeated, and the amount (ES Flux [$\mu g/cm^2/hr$]) of the drug to be permeated from a given area of the skin in a fixed time period was calculated from the drug concentration (measured by HPLC) of the recovered solution. From the results of Table 3, the sample of Comparative Example 4 was excluded from the permeability test. The results are shown in Fig. 1.
As is clear from Fig. 1, the patch preparations obtained in Examples 1 - 3 reached a given release amount in about 10 hr after adhesion, and almost constant permeability was obtained in the permeability test up to 72 hr after adhesion. In contrast, the patch preparations prepared in Comparative Examples 1 - 3 showed the maximum release amount within 10 hr after adhesion, and the drug permeability decreased after 12 hr from adhesion or showed remarkable decrease tendency. From the above-mentioned test results, it was confirmed that the patch preparation of the present invention releases a poorly soluble drug continuously and stably even after adhesion to the skin for a long time.

Experimental Example 3 (Evaluation of crystal formation) The crystal formation of estradiol in a patch preparation was evaluated as follows. (name of apparatus) digital microscope, VHX-600 manufactured by KEYENCE (Test method) Polarized lens were combined, and the presence or absence of crystals was confirmed by visual evaluation according to a permeation method. All patch preparations obtained in Examples 1 - 3 of the present invention could retain the poorly soluble drug without crystal precipitation. (Test conditions) room temperature for three months, sample shape 2 cm x 2 cm

From the above-mentioned results, the effect of the present invention was demonstrated, since the adhesive layer contained a poorly soluble drug, the first, second and third organic liquid components, and crosslinked polyvinylpyrrolidone.

While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.

**Claims**

1.  A patch preparation having an adhesive layer on at least one surface of a support,
    wherein said adhesive layer comprises an adhesive, a poorly soluble drug, a first organic liquid component, a second organic liquid component, a third organic liquid component and a crosslinked polyvinylpyrrolidone, the solubility of said poorly soluble drug in said first organic liquid component is not less than the solubility of said poorly soluble drug in said second organic liquid component, and the solubility of said poorly soluble drug in said second organic liquid component is not less than the solubility of said poorly soluble drug in said third organic liquid component.

2.  The patch preparation of claim 1, wherein said first organic liquid component is a first polyvalent alcohol, said second organic liquid component is a fatty acid ester of a second polyvalent alcohol having at least one hydroxyl group of said second polyvalent alcohol, and said third organic liquid component is a monovalent alcohol.

3.  The patch preparation of claim 2, wherein the fatty acid ester of said second polyvalent alcohol is a fatty acid monoester of a divalent alcohol, or a fatty acid mono- or diester of a trivalent alcohol.

4.  The patch preparation of claim 1, 2, or 3 wherein said adhesive layer comprises 0.1 - 15 parts by weight of said second organic liquid component relative to 100 parts by weight of the adhesive.

5.  The patch preparation of any one of the preceding claims,
    wherein said adhesive is a rubber adhesive.

6.  The patch preparation of claim 5, wherein said rubber adhesive comprises the first rubber elastomer having a viscosity average molecular weight of 1,600,000 - 6,500,000 and the second rubber elastomer having a viscosity average molecular weight of 40,000 - 85,000.

7.  The patch preparation of any one of the preceding claims,
    wherein the melting point of said poorly soluble drug is not less than 100°C.

8.  The patch preparation of claim 7, wherein said poorly soluble drug is a drug having log Pow of 0.5 - 5.5.

FIG. 1

Europäisches
Patentamt

European
Patent Office

Office européen
des brevets

## EUROPEAN SEARCH REPORT

Application Number

EP 08 25 3376

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 352 649 A (NITTO DENKO CORP [JP]) 15 October 2003 (2003-10-15) * abstract * * paragraph [0008] * ----- | 1-8 | INV. A61K9/70 A61K47/10 A61K47/32 |
| A | US 5 462 746 A (WOLTER KARIN [DE] ET AL) 31 October 1995 (1995-10-31) * the whole document * ----- | 1-8 | |
| A | DE 101 03 860 A1 (LOHMANN THERAPIE SYST LTS [DE]) 14 August 2002 (2002-08-14) * the whole document * ----- | 1-8 | |

**TECHNICAL FIELDS SEARCHED (IPC)**

A61K

The present search report has been drawn up for all claims

| Place of search | Date of completion of the search | Examiner |
|---|---|---|
| Munich | 12 March 2009 | Felder, Christian |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 08 25 3376

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2009

| Patent document cited in search report | | Publication date | Patent family member(s) | | Publication date |
|---|---|---|---|---|---|
| EP 1352649 | A | 15-10-2003 | AT | 316370 T | 15-02-2006 |
| | | | CA | 2425231 A1 | 12-10-2003 |
| | | | DE | 60303326 T2 | 12-10-2006 |
| | | | DK | 1352649 T3 | 06-03-2006 |
| | | | ES | 2252573 T3 | 16-05-2006 |
| | | | JP | 2003300868 A | 21-10-2003 |
| | | | US | 2003203697 A1 | 30-10-2003 |
| US 5462746 | A | 31-10-1995 | NONE | | |
| DE 10103860 | A1 | 14-08-2002 | AT | 395050 T | 15-05-2008 |
| | | | DK | 1355636 T3 | 08-09-2008 |
| | | | WO | 02060418 A1 | 08-08-2002 |
| | | | EP | 1355636 A1 | 29-10-2003 |
| | | | ES | 2307737 T3 | 01-12-2008 |
| | | | JP | 4160394 B2 | 01-10-2008 |
| | | | JP | 2004516916 T | 10-06-2004 |
| | | | US | 2004071764 A1 | 15-04-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8104625 A
- JP 2503095 B
- JP 7098744 B